# EUROPEAN PATENT APPLICATION

(11) **EP 3 533 484 A1**
(43) Date of publication of application: **04.09.2019**
(21) Application number: 18159564.6
(22) Date of filing: 01.03.2018
(51) Int. Cl.: A61M 25/00, A61M 29/00, A61M 25/10

(54) **CONTROLLED PRESSURE REPERFUSION CATHETER**

(71) Applicant: Özer, Mehmet Ali, 34734 Istanbul (TR)
(72) Inventor: Özer, Mehmet Ali, 34734 Istanbul (TR)
(74) Representative: Sevinç, Erkan

(57) **Abstract**

A reperfusion catheter for controlled reperfusion in an occluded artery is disclosed. The reperfusion catheter comprises a tubular perfusion catheter (12) having a proximal end and a distal end and a lumen defined in between said proximal and distal ends. The catheter (12) has a dilatator which is sized and shaped to move telescopically in the lumen of said perfusion catheter. The tubular perfusion catheter has at least one perfusion port (16) which is formed in the shell of the tubular perfusion catheter (12) and which establishes fluid communication in between the proximal end and the lumen of the perfusion catheter. The at least one perfusion port (16) extends in the longitudinal direction of the perfusion catheter such that, in use, full or partial withdrawal of the dilatator out of the lumen of the perfusion catheter permits controlled flow of blood through said perfusion port (16) and out of the distal end of the tubular perfusion catheter (12).

## Description

### FIELD OF THE INVENTION

The present invention relates to a new catheter for controlled release of blood flow in an occluded artery. The catheter is particularly useful in the treatment of patients suffering from acute ST-segment elevation myocardial infarction (STEMI) caused by sudden occlusion of an epicardial coronary artery. Nevertheless, the new catheter may be useful in the treatment of occluded blood vessels in other parts of the body, such as vessels feeding the brain. The present invention also discloses a system comprising the catheter of the invention and a method of treatment of any occluded artery supplying a severely ischemic area

### BACKGROUND OF THE INVENTION

Acute ST-segment elevation myocardial infarction (STEMI) caused by sudden occlusion of an epicardial coronary artery can be regarded as one of the most important life-threatening pathology in the field of clinical cardiology. Re-opening of the occluded epicardial coronary artery by timely Primary Percutaneous Coronary Intervention PPCI) is widely accepted as the most effective treatment modality for patients presenting with an acute STEMI. However, successful reopening of the occluded infarct-related artery by PPCI does not immediately terminate progressive microvascular injury at the subtended myocardial territory. This ongoing nature of microvascular injury leading to a progressive myocardial malperfusion increases final myocardial infarct size, and negatively effects cardiac structure and function, therefore patients' outcome. Despite successful PPCI, mortality (15%) and particularly post-MI morbidity (heart failure) still remain significant at 1-year. Nearly half of the all new heart failure in patients under 75 years old linked to STEMI.

Obviously, re-establishing complete and sustained epicardial patency by PPCI in a timely manner is the most critical step in salvaging ischemic myocardium from impending infarction. However, prompt restoration of coronary flow by mechanical re-opening the occluded infarct-related artery using current angiographic therapeutic intervention techniques (PPCI) can itself paradoxically induces coronary microvascular injury and may substantially contribute to magnitude of the cardiomyocyte loss at the myocardial area at risk. This disappointing course has been partly attributed to the potential detrimental effects of reperfusion itself, namely 'reperfusion injury', which is thought to cause further propagation of cardiomyocyte loss in the subtended myocardial territory. Reperfusion injury is a complex phenomenon having various aspects needs to be more clarification and solution, reperfusion related microvascular injury as one of these aspects, also needs a new understanding and approach for a new and better therapeutic achievement.

Following successful reopening of the epicardial coronary artery by PPCI, microvascular (intraluminal) obstruction (mainly thought to be caused by distal thromboembolization due to manipulation/liberation of the epicardial fresh thrombus during PPCI), in-situ thrombosis and extravascular compression of the microcirculation by myocardial edema and intramyocardial hemorrhage (IMH) can contribute to microvascular injury and limit reperfusion flow in the subtended myocardial territory.

Notably, large-scale manual thrombectomy trials, which targeted lessening the risk of distal thromboembolization via aspiration of the fresh intraluminal thrombus causing coronary occlusion, aiming to prevent microvascular obstruction during PPCI repeatedly failed to show any clinical benefit despite they were successful in retrieving thrombotic material responsible for acute occlusion from the coronary artery lumen. Therefore, for now, any therapeutic intervention aiming to prevent microvascular obstruction by reducing distal embolization risk during PPCI procedure does not seem to yield any clinical benefit.

Methods, such as local adjunctive selective fibrinolytics infusion, aiming to dissolve in-situ microvascular thrombus that may develop during acute coronary occlusion needs to be elucidated.

On the other hand, external compression of the microcirculation by myocardial edema and IMH developed in the surrounding myocardium after reperfusion, may substantially contribute to post-PPCI microvascular injury and myocardial malperfusion. Almost all patients undergoing reperfusion by PPCI developed edema at different intensities. In addition, it was reported that approximately 50% of the patients undergoing PPCI had IMH.

During total occlusion of an epicardial coronary artery, depending on the duration and severity of the ischemia, hypoxia-induced capillary increased permeability and disruption results in microvascular leakage, which is the central anatomical substrate underlying myocardial edema and hemorrhage occurring after establishment of reperfusion. Concurrently during epicardial occlusion, due to both severely increased myocardial demand (adaptive vasodilator response) and partly to ischemic insult (decreased contractile function), arteriolar sphincters, which regulate myocardial blood flow, are supposed to lose their regulatory vasoconstrictor function. Therefore, subtended microvascular segments are deprived of the protection of pressure-regulating resistance arterioles at the time when reperfusion abruptly restarts by PPCI. Upon reperfusion established promptly by PPCI, before coronary autoregulation is restored, leaky and unguarded microcirculation is exposed to a pressure burst due to abruptly increased capillary hydrostatic pressure, which causes interstitial myocardial edema and IMH. Therefore, sudden exposure of this unguarded and leaky distal microcirculation to an extremely high distal intracoronary pressure by abrupt reperfusion accomplished by immediate stenting (PPCI) may generate a barotrauma-like effect on the infarct related area. So, uncontrolled and sudden rise in distal intracoronary pressure by successful PPCI, before coronary autoregulatory function in the reperfused myocardial territories is recovered, may promote severe myocardial edema and IMH which seem to be the main determinants of post - PPCI microvascular injury.

It is known that after reopening of the occluded epicardial coronary artery, following initial hyperemic flow, myocardial blood flow in the area at risk rapidly and progressively declines and gets stabilized approximately within 30 minutes. This finding implies that, in spite of the initial ischemic insult, coronary auto-regulation in the reperfused myocardial territory recovers and controls myocardial blood flow. Nevertheless, this recovery of the malfunctioned pre-arteriolar sphincters requires some time after reperfusion was achieved. Therefore, full restoration of perfusion pressure distal to the infarct related artery by immediate stenting (PPCI) then fail to induce an immediate adaptive autoregulatory response (compensatory vasoconstriction) in the arterioles of the related microvascular territory, resulting in an inappropriately increased pressure in the already damaged microcirculation leading to edema and IMH.

Routine PPCI comprises direct stent implantation or it is preceded by a balloon angioplasty. Although this is the most beneficial method for salvaging myocardial tissue, it also has above-mentioned consequences which limit its efficacy. Up to now, all adjunctive mechanical intervention strategies (mechanical and manual thrombectomy devices, distal and proximal protection devices or mesh covered stents) implemented to limit reperfusion-related microvascular obstruction by reducing the distal embolization risk in PPCI setting repeatedly failed to show any clinical benefit. In addition, adjunctive pharmacological interventions, mainly intracoronary delivery of glycoprotein IIb-IIIa inhibitors and vasoactive agents, were shown to be ineffective in limiting microvascular injury during PPCI.

Routine primary percutaneous coronary interventions (PPCI) for ST-elevation acute myocardial infarction (STEMI) includes stent implantation either directly or following balloon angioplasty. In limited number of cases, manual thrombectomy might be performed prior to stent implantation. Critically, after abrupt and complete removal of epicardial occlusion by stent implantation following balloon angioplasty or thrombectomy resulting in equalization of proximal and distal intracoronary pressures in the infarct related artery exposes the subtended microcirculation to a high and uncontrolled pressure. PPCI by current devices and methods leads to a rapid reflection of arterial pressure to the unguarded (open) and fragile microvasculature. This consequence of routine PPCI substantially exaggerate the edema and not rarely IMH, both of which can contribute to microvascular damage by external compression. During PPCI, angioplasty even with a small balloon or thrombectomy alone may result in an uncontrolled, abrupt and complete/high pressure reperfusion, which can cause uncontrolled pressure burst in already injured capillary bed. Therefore, using conventional techniques, distal pressure and flow cannot be guaranteed throughout the PPCI procedure.

In brief, although at present, PPCI applied with conventional devices and methods is the most effective treatment modality for lessening of ischemic injury in STEMI patients, it does not prevent from, even contrary augments microvascular injury, therefore needs to be improved.

Intracerebral hemorrhage and/or edema developed immediately after stenting of a high-grade carotid stenosis, namely mostly fatal hyperperfusion syndrome, most likely shares the same mechanism with IMH and edema formation. In order to maintain cerebral blood flow during long-lasting hypoperfusion period, cerebral autoregulation maximally dilates the arterioles distal to the severely stenosed carotid artery. In conjunction with this adaptive vasodilatory response, the autoregulatory function of the arteriolar sphincters is impaired in this severely ischemic cerebral tissue. Therefore, cerebral arteriolar malfunction in the subtended microcirculatory territory results in an insufficient vasoconstrictor response to the suddenly increased intravascular pressure abruptly reestablished by stenting of nearly occluded carotid arteries. Consequently, by a similar mechanism as in STEMI patients, suddenly increased intracapillary hydrostatic pressure disrupts the tight junctions of the capillary endothelial cells and causes intracerebral hemorrhage. This common background strongly suggests the pivotal role of abrupt re-opening of an occluded artery in the development of hemorrhage and/or edema in the subtended microvascular territory.

### OBJECTS OF THE PRESENT INVENTION

The primary object of the present invention is to eliminate the drawbacks outlined in the background of invention.

Another object of the present invention is to provide a catheter system which allows controlled and gradual reperfusion in the microvascular territory supplied by a large feeder vessel to prevent abrupt increase in distal intra-vascular and consequently capillary hydrostatic pressure during initial reperfusion (reactive hyperemia) phase.

A further object of the present invention is to provide a catheter system which allows controlled release of blood flow such that reperfusion pressure in the arterioles can be controlled until their protective vasoconstrictor functions can be restored.

Yet another object is to provide a catheter system which limits microvascular injury, lessens leakage from the capillaries and therefore reduces interstitial hemorrhage and/or edema formation.

Other objects of the present invention will become apparent from accompanied drawings and description.

### SUMMARY OF THE INVENTION

In order to achieve the objects of the invention, the present invention proposes a catheter for controlled reperfusion in an occluded artery. The catheter comprises a tubular perfusion catheter having a proximal end and a distal end and a lumen defined in between said proximal and distal ends. The catheter has a dilatator which is sized and shaped to move telescopically in the lumen of said perfusion catheter. Distinguishingly, the tubular perfusion catheter has at least one perfusion port which is formed in the shell of the tubular perfusion catheter and which establishes fluid communication in between the proximal end and the lumen of the perfusion catheter. The at least one perfusion port is fully blocked by the dilatator when the dilatator is fully inserted in the lumen of the perfusion catheter. The at least one perfusion port extends in the longitudinal direction of the perfusion catheter such that, in use, full or partial withdrawal of the dilatator out of the lumen of the perfusion catheter permits controlled flow of blood through said perfusion port and out of the distal end of the tubular perfusion catheter.

The perfusion port may be formed by a plurality of perfusion holes which are spaced apart from each other in the longitudinal direction of the tubular perfusion catheter. Alternatively, the perfusion port may be formed by one or more slits which extend parallel to the longitudinal direction of the tubular perfusion catheter.

The reperfusion catheter may comprise an inflatable balloon wrapped around the outer periphery of the perfusion catheter near its distal end. A balloon channel may be formed on the shell of the perfusion channel for inflating or deflating the balloon.

The dilatator may have an opening which connects its proximal and distal ends and which accommodates a guide wire. The cross sectional area of opening may be at least 25% more than cross sectional area of the guide wire so that an injectate may be conveyed around the guide wire from the proximal end of the dilatator to its distal end.

The catheter may comprise a pressure sensor located at the distal end of the perfusion catheter such that pressure of the blood flow thought the distal end of the tubular perfusion catheter can be monitored in real time. Likewise, the catheter may comprise a Doppler sensor located at the distal end of the perfusion catheter such that velocity of the blood flow thought the distal end of the tubular perfusion catheter can be monitored.

In another aspect, the dilatator or the perfusion catheter or both may have parking markers for indicating the longitudinal position of the dilatator within the lumen of the perfusion catheter. The dilatator may have a tapered tip at its distal end for facilitating penetration in a occluded/severely stenosed vessel. The catheter may comprise a guiding catheter for accommodating the perfusion catheter and components.

The catheter may comprise a pressure sensor line for conveying data obtained from the pressure sensor. Likewise, the catheter may comprise a Doppler sensor line for conveying data obtained from the Doppler sensor.

In another aspect, the dilatator may have an opening which connects its proximal and distal ends and which accommodates a guide wire. The cross sectional area of opening is at least 25% more than cross sectional area of the guide wire so that an injectate may be conveyed around the guide wire the from the proximal end of the dilatator to its distal end. The injectate may be one or more therapeutic agents in liquid form which needs to be infused through the distal end of the dilatator.

In yet another aspect, the dilatator may have one or more flexible protrusions which fit into cavities formed on the perfusion catheter such that the dilatator can be detachably fixed in a plurality of parking positions in the longitudinal direction of the perfusion catheter. Likewise, the protrusions may be formed on the perfusion catheter rather than the dilatator, in which case the cavities may be formed on the dilatator.

The present invention also discloses a system comprising the catheter of Claim 1, a microcontroller for receiving and processing the real time data from the pressure sensor and/or the Doppler sensor and a screen for displaying the received data.

The present invention also discloses a method for treating an occluded blood vessel, said method comprising the steps of;
- penetrating a guiding wire through the occlusion
- advancing/penetrating a dilatator over the guide wire through the occlusion
- advancing a tubular perfusion catheter through the occlusion such that the dilatator is accommodated in the lumen of the perfusion catheter
- optionally inflating a balloon within the stenosis of the occluded blood vessel,
- moving dilatator longitudinally within the lumen of the tubular perfusion catheter such that a controlled reperfusion is established through perfusion holes which are spaced apart from each other in the longitudinal direction of the tubular perfusion catheter.

### BRIEF DESCRIPTIONS OF THE DRAWINGS

Accompanying drawings are given solely for the purpose of exemplifying the catheter system proposed by the present invention whose advantages over prior art were outlined above and will hereinafter be explained in detail.

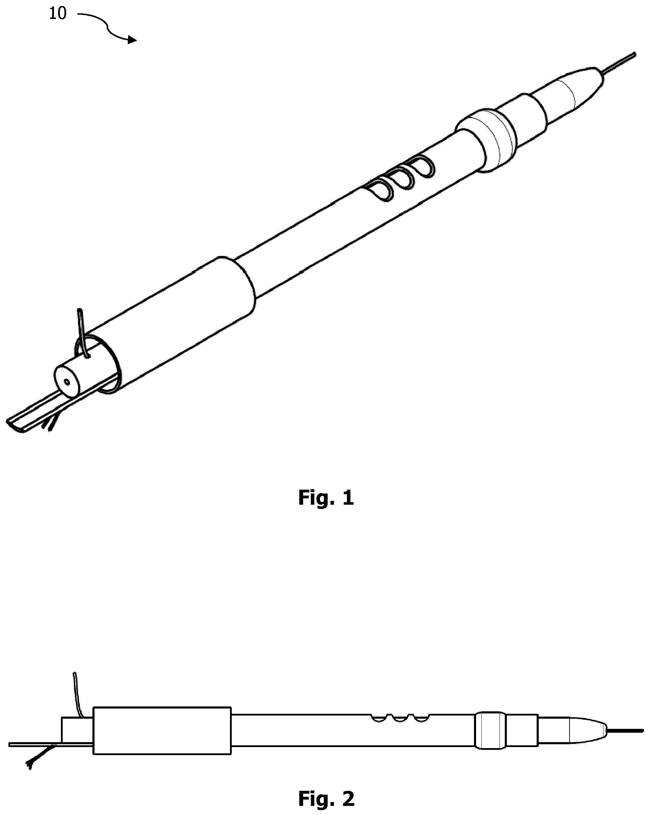

Fig. 1 shows perspective view of a catheter according to the present invention,
Fig. 2 shows the side view of the catheter of Fig. 1,
Fig. 3A shows two-dimensional view of an artery having no occlusion,
Fig. 3B shows occluded segment of the artery,
Fig. 3C shows the guide wire of the catheter passing through the culprit lesion shown in Fig. 3B,
Fig. 3D shows the dilatator of the catheter advancing across the occluded segment of the artery,
Fig. 3E shows perfusion catheter moving distal to the occluded segment over the dilatator,
Fig. 3F shows the catheter in a state where the balloon is inflated in the stenosis
Fig. 3G shows the catheter in a state where the dilatator is withdrawn back towards the proximal of the perfusion holes and blood flows first into the lumen of the perfusion catheter and then to the distal part of the occluded artery,

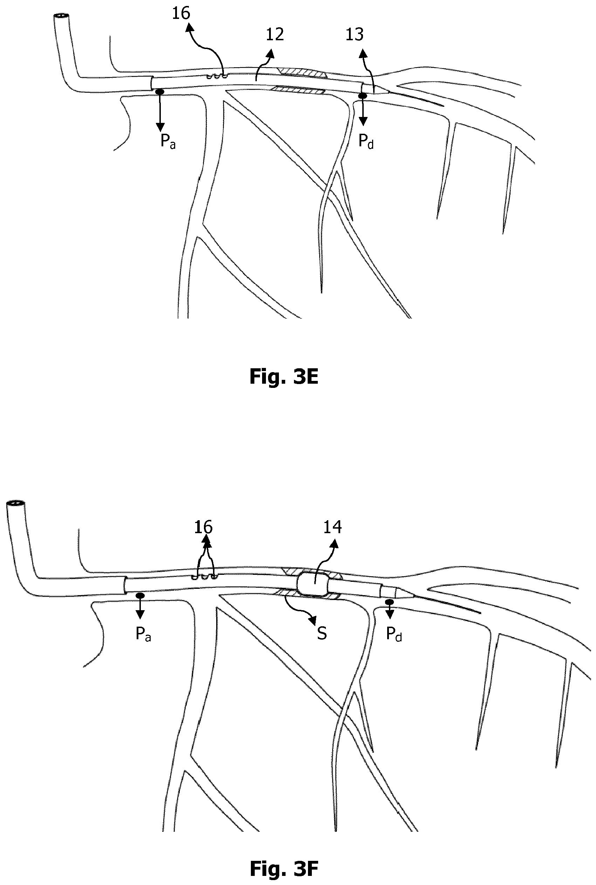

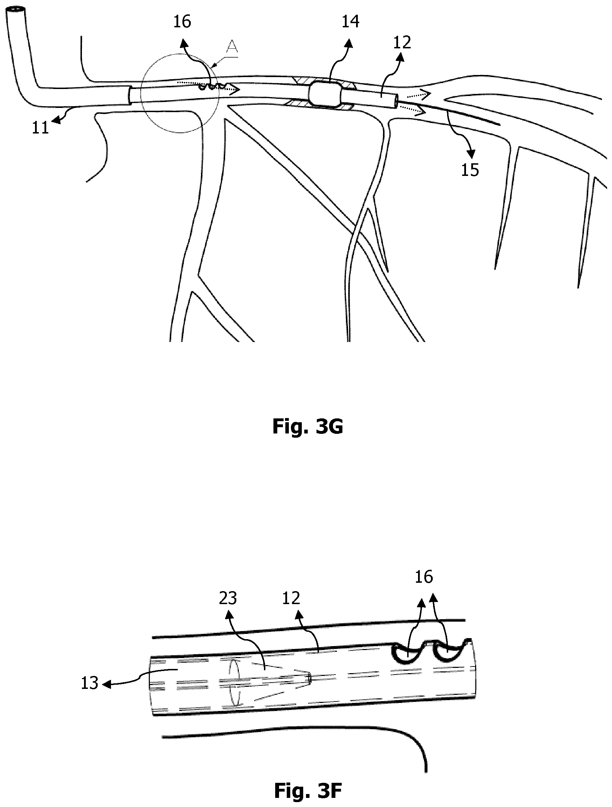

Fig. 3F is a close-up view of detail A of Fig. 3G,

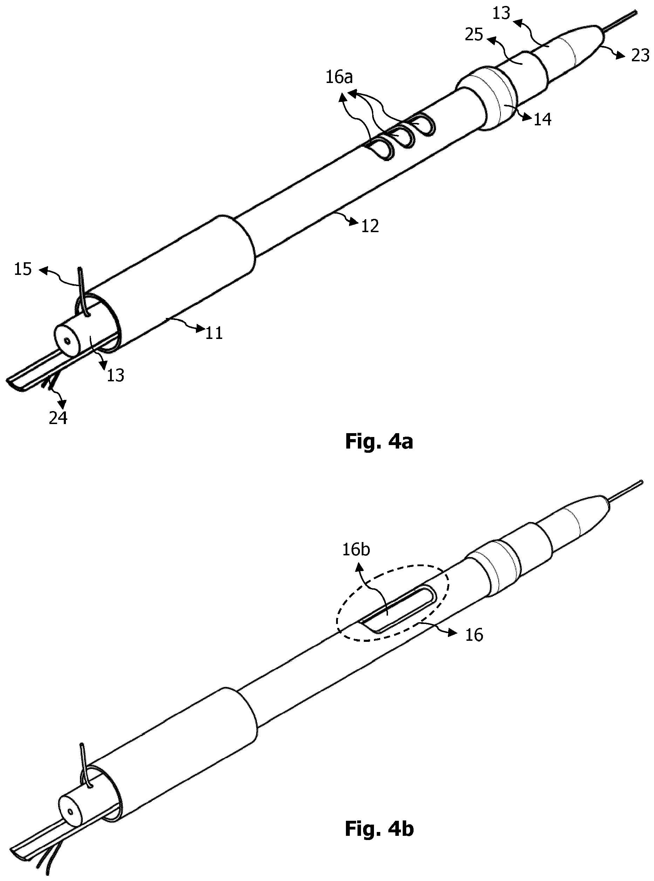

Fig. 4a shows perspective view of a catheter according to the present invention,
Fig. 4b shows perspective view of a different embodiment of the catheter according to the present invention,

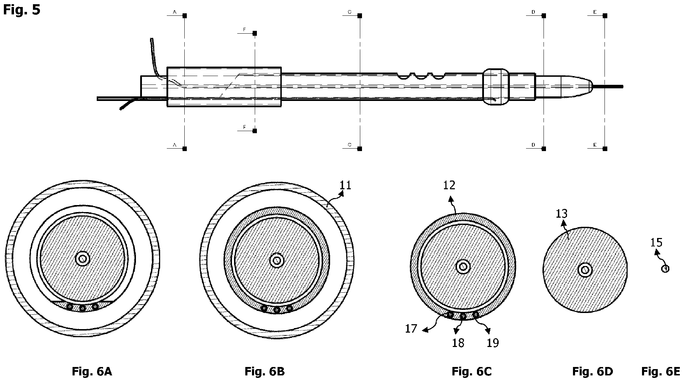

Fig. 5 depicts the side of the catheter of Fig. 4 showing the sectional lines,
Fig. 6A shows the A-A cross sectional view of Fig. 5,
Fig. 6B shows the F-F cross sectional view of Fig. 5,
Fig. 6C shows the C-C cross sectional view of Fig. 5,
Fig. 6D shows the D-D cross sectional view of Fig. 5,
Fig. 6E shows the E-E cross sectional view of Fig. 5,

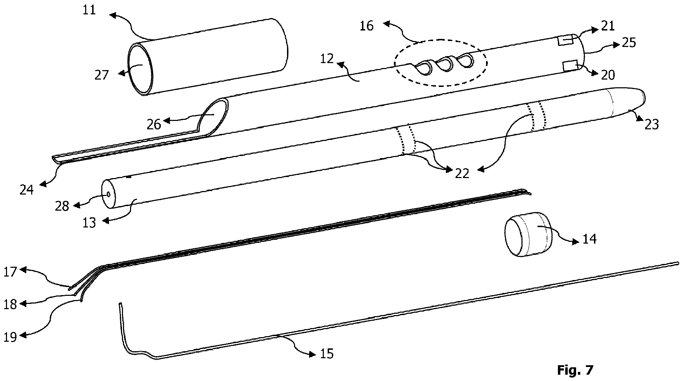

Fig. 7 shows the exploded view of the catheter according to the present invention,

### DETAILED DESCRIPTION OF THE INVENTION

Proposed device aims to limit microvascular injury. Particularly, this invention includes a new set of devices enabling application of a new method to successfully limit reperfusion-related microvascular injury mainly by preventing uncontrolled/sudden rise in pressure in the reperfused microvascular territory. As of the application date, no devices are available to limit microvascular injury developing in scenarios such as invasive angiographic therapeutic procedures performed to re-open acutely occluded coronary arteries or to open very severely stenotic carotid arteries so far. Using this proposed device, a catheter system comprising specialized perfusion and optional sensing parts and control console, will provide to monitorize and control of reperfusion and may prevent edema and hemorrhage formation developed after opening of the occluded or severely stenotic vessels supplying the related organ systems (heart or brain) by therapeutic angiographic interventions such as reperfusion by stenting. This technique will permit real time operator feedback for therapeutic decision-making and so create a system feasible for interventional procedures.

Hereinafter, preferred embodiments of the present invention will be described in detail with reference to the accompanying drawings. The table of reference numerals used in the appended drawings is as follows;
- 10: catheter
- 11: guiding catheter
- 12: perfusion catheter
- 13: dilatator
- 14: balloon
- 15: guide wire
- 16: perfusion port
- 16a: perfusion hole
- 16b: perfusion slit
- 17: balloon channel
- 18: Doppler sensor line
- 19: pressure sensor line
- 20: pressure sensor
- 21: Doppler sensor
- 22: parking marker
- 23: tapered tip
- 24: proximal end
- 25: distal end
- 26: lumen of the perfusion catheter
- 27: lumen of the guiding catheter
- 28: lumen of the dilatator
- S:: occluded/stenosed segment

The catheter-based device according to the present invention allows a gradual, controlled, low pressure reperfusion and make possible to assess, monitor and quantify the treatment effect during the therapeutic interventional procedure and guide the therapy in real time.

In order to achieve the objects of the invention, the present invention proposes a reperfusion catheter (10) for controlled reperfusion in an occluded artery, said catheter (10) comprising a tubular perfusion catheter (12) having a proximal end (24) and a distal end (25) and a lumen (26) defined in between said proximal and distal ends (24,25), and a dilatator (13) which is sized and shaped to move telescopically in the lumen (26) of said perfusion catheter (12) Distinguishingly, the tubular perfusion catheter (12) has at least one perfusion port (16) which is formed in the shell of the tubular perfusion catheter (12) and which establishes fluid communication in between the proximal end (24) and the lumen (26) of the perfusion catheter (12). Said at least one perfusion port (16) is fully blocked by the dilatator (13) when said dilatator is fully inserted in the lumen (26) of the perfusion catheter (12). Said at least one perfusion port (16) extends in the longitudinal direction of the perfusion catheter (12) such that, in use, full or partial withdrawal of the dilatator (13) out of the lumen (26) of the perfusion catheter (12) permits controlled flow of blood through said perfusion port (16) and out of the distal end (25) of the tubular perfusion catheter (12).

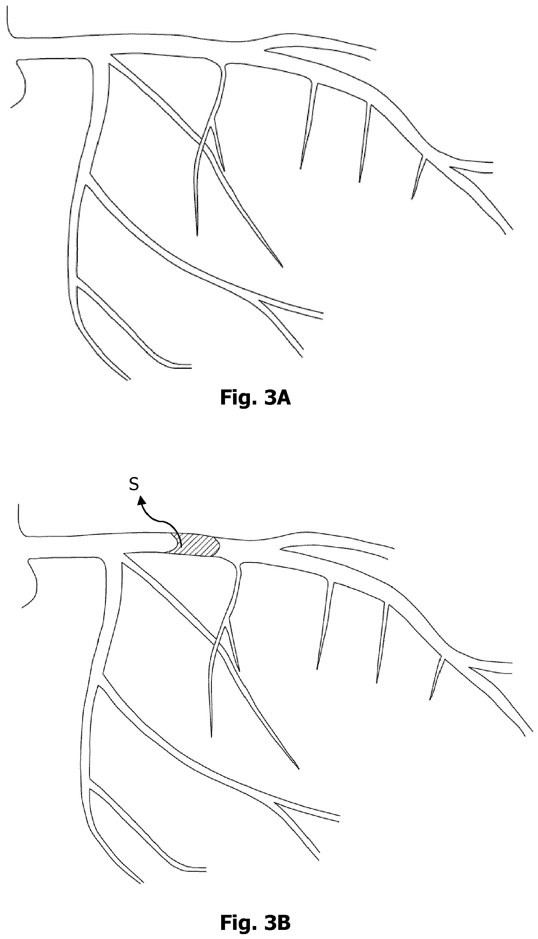

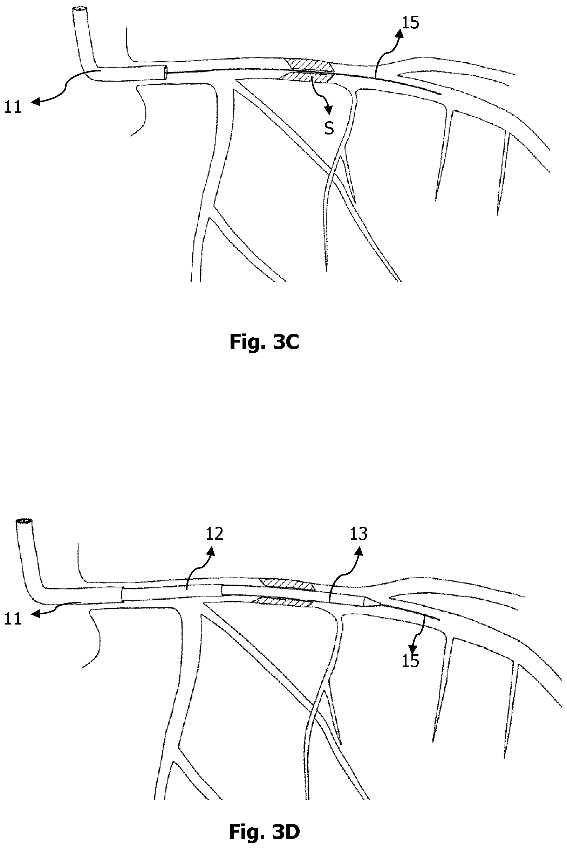

Two-dimensional view of an artery having no infarction is illustrated in Fig. 3A. However, Fig. 3B shows an occluded segment of an artery. According to the novel therapeutic method of the present invention, the new catheter (10) is navigated in the occluded artery as shown in Fig. 3C. A guide wire (15) is pushed forward towards the stenosis and passes through the culprit lesion as illustrated in Fig. 3C. Later on, the dilatator (13) is pushed forward towards the stenosis and passes through the culprit lesion as shown in Fig. 3D. While passing through the occlusion (S), the dilatator (13) is guided on the guide wire (15) which penetrated the occlusion (S) in the previous step. The dilatator has a tapered tip (23) on its distal end in order to facilitate its movement in the longitudinal direction of the dilatator (12).

Next step of the treatment is advancing the perfusion catheter (12) through the culprit lesion as illustrated in Fig. 3E. While penetrating through the stenosis, the perfusion catheter (12) is guided on the dilatator (13). Once the perfusion catheter (12) fully penetrates through the stenosis, the distal end (25) of the lumen (26) of the perfusion catheter (12) is located downstream the stenosis (S), whereas the perfusion holes (16a) remain upstream the stenosis (S), i.e. close to the proximal end of the perfusion catheter (12). As illustrated in Fig. 3E, the blood pressure on the proximal of the occlusion (S) is named as Pₐ obtained from guiding catheter (11), whereas the blood pressure on the distal of the occlusion obtained from pressure sensor (20) located at the distal end of the catheter, named as P_{d}. At this point of treatment Pa is substantially larger than Pd since the stenosis blocks the flow in the artery.

With reference to Fig. 3E, if the blood pressure downstream the stenosis is found to be close to the blood pressure upstream the stenosis, i.e. Pd ≈ Pa, this would mean that a high-pressure reperfusion has started unintentionally. Complete or high-pressure reperfusion may occur unintentionally just by introducing the system in to the freshly occluded infarct related artery if there is any peri-catheter antegrad flow. In this case, the balloon (14) mounted on perfusion catheter (12) is inflated, as shown in Fig. 3F, in order to prevent microvascular reperfusion mediated barotrauma. The balloon (14) may be inflated by applying a pressurized fluid (such as diluted contrast agent) through the balloon channel (17) located on the proximal end of the perfusion catheter (12). The balloon channel (17) extends in between the proximal end (24) of the perfusion catheter (12) and the balloon (14). The balloon (14) is located near the distal end (25) of the perfusion catheter (12). Once the balloon (14) is inflated the pressure (P_{d}) downstream the stenosis reduces substantially. As in the case of immediate stenting, the downstream pressure would be almost equal to the considerably high upstream pressure, the patient would suffer microvascular injury leading to a progressive myocardial malperfusion.

At the next step of the treatment, distal reperfusion is initiated by pulling the dilatator (13) towards the proximal end (24) of the perfusion catheter (12). This maneuver opens one or more of the plurality of perfusion holes (16a) proximal to inflated balloon (14) and initiates controlled flow through the distal end (25) of the perfusion catheter (12). Antegrade blood flow enters into the lumen (26) of the perfusion catheter (12) through the plurality of perfusion holes (16a) located proximally to the balloon (14). The term antegrade blood flow is used to mean the flow upstream the occlusion/stenosis. The inflow pass through the catheter lumen (26) and leave the perfusion catheter (12) from the distal end (25). In this case, distal perfusion become fully controllable and adjustable since the operator may decide on the amount of inflow by adjusting dilatator and perfusion hole orientation, and thus the downstream blood pressure Pd. The more the dilatator (13) is moved back to the proximal end of the perfusion catheter (12), means the more rows of perfusion holes (16a) are unblocked by the dilatator (13). The dilatator (13) is sized and shaped to fill in the lumen (26) of the perfusion catheter (12). Once the dilatator (13) is withdrawn towards the proximal end (24) of the perfusion catheter (12), the plurality of rows of perfusion holes (16a) formed on the shell of the perfusion catheter (12) becomes in blood communication with the lumen (26) of the perfusion catheter (12). As the rows of perfusion holes (16a) are spaced apart from each other in the longitudinal direction of the perfusion catheter (12), the more the dilatator is moved towards the proximal end (24) of the catheter, the more number of perfusion holes (16a) will connect the upstream blood flow with the lumen (26) and hence, the distal end (25) of the perfusion catheter (12). The arrows shown in broken lines in Fig. 3G depicts the flow path of upstream blood flow first in the lumen (26) and then towards downstream the occlusion.

Distal vessel and microvascular bed receive this flow generating a filling pressure. Since antegrade flow is fully bounded to the catheter system (10), distal perfusion pressure (P_{d}) is determined by the magnitude of the blood flow entering into the perfusion catheter (12) from the plurality of perfusion holes (16a). Advancing forward or pulling back the dilator (13) inside the perfusion catheter (12) makes perfusion holes (16a) partially or fully, open or closed. As shown in Fig. 3G, the dilatator (13) is fully moved back towards the proximal end (24) of the catheter (12) and as shown in Fig. 3F, which is a close close-up view of detail A of Fig. 3G, all rows of the plurality of the perfusion holes (16a) becomes unblocked, resulting in full upstream flow entering into the lumen (26) of the perfusion catheter (12). Distal flow determined by the number of the open perfusion holes (16a), adjustable by changing dilatator-perfusion hole orientation, will therefore generate a distal pressure (Pd) that fills distal vessels and microcirculation. Using this approach uncontrolled or unintentional rise in distal pressure can be prevented. This alone prevents or at least limits the microvascular damage downstream the stenosis.

The proposed catheter system will provide pressure and flow control beyond the culprit lesion in early reperfusion phase throughout the PPCI and carotid artery stenting (CAS) procedures. Fully controlled, low-pressure perfusion achieved by this catheter-based device (10) will allow enough time to pressure regulating arterioles until their adaptive vasoconstrictor response get recovered. Meanwhile, sustained and reliable flow to distal microcirculation will be provided in controlled pressures by the perfusion catheter (12). This will allow the operator to follow patient until the time when autoregulatory function of the arterioles in the reperfusion area get recovered and protects distal capillary bed from the detrimental effect of the promptly established inappropriately high pressures. The inventors have visualized that the recovery time is around 30 minutes. Thereafter, equalization of the distal perfusion pressure to proximal intravascular pressure via total elimination of occlusion by stent implantation (full restoration of reperfusion) can safely be possible. This approach can therefore substantially limit to edema and hemorrhage formation at the reperfused tissue.

As shown in Fig. 4a, the perfusion port (16) may be formed by a plurality of perfusion holes (16a) which are spaced apart from each other in the longitudinal direction of the tubular perfusion catheter (12). Alternatively, the perfusion port (16) may be formed by one or more slits (16b) which extend parallel to the longitudinal direction of the tubular perfusion catheter (12) as depicted in Fig. 4b. For ease of understanding, the perfusion port (16) will be assumed to be formed by a plurality of perfusion holes (16a) in the remainder of this text.

The catheter (10) according to the present invention has been illustrated to have three perfusion holes (16a) as depicted in Fig. 4a. While these perfusion holes (16a) have been shown to be decently aligned in the longitudinal direction of the perfusion catheter (12), the perfusion holes (16a) may be distributed around the periphery of the perfusion catheter (12) and may be formed in a plurality of rows. This latter means that there might be a plurality of perfusion holes (16a) in the exactly same longitudinal position of the perfusion catheter (12). The size of the holes may be selected depending on the number of holes (16a) in each of the longitudinal position of the perfusion catheter. The perfusion holes located closer to the distal end of the perfusion catheter (12) may have a smaller size as compared to the holes (16a) located farther to the distal end in order to allow the operator to make fine tuning in the downstream pressure (P_{d}).

Fig. 5 depicts the side view of the catheter (10) according to the present invention showing the sectional lines. Fig. 6A to 6E show the cross-sectional views marked in Fig. 5, in which the dilatator (13) was fully inserted in the perfusion catheter (12) and all perfusion holes (16a) were blocked. As shown in Fig. 6C, there shall be a small gap in between the dilatator (13) and the perfusion catheter (12) so that the dilatator (13) shall smoothly move telescopically in the lumen (26) of the perfusion catheter (12). The catheter (10) may also comprise a guiding catheter (11) having a lumen (27) for accommodating the perfusion catheter (12) other components contained in its lumen (26).

The reperfusion catheter (10) may comprise an inflatable balloon (14) wrapped around the outer periphery of the perfusion catheter (12) near its distal end. The controlled reperfusion would nevertheless be established in the absence of the inflatable balloon (14). However, the balloon (14) may be useful in eliminating undesired reperfusion which may start due to the vascular intervention. As illustrated in Fig. 7, the shell of the perfusion catheter may have a balloon channel (17) for inflating or deflating the balloon (14).

As shown in Fig. 7, the dilatator (13) or the perfusion catheter (12) or both may have markings (22) which indicate the longitudinal position of the dilatator (13) with respect to the lumen (26) of the perfusion catheter (12). Using the marking, the operator would be able to easily understand and adjust the position of the dilatator (13) and hence, the distal pressure P_{d} downstream the stenosis.

The dilatator (13) has a central lumen (28) for accommodating the guide wire (15). As shown in Fig. 7, the lumen (28) extends fully from the proximal end until the distal end of the dilatator (13). The size of the lumen (28) shall be sufficient to accommodate the guide wire (15) and allow its telescopic movement.

In yet another aspect, the dilatator (13) may have one or more flexible protrusions (not shown) which fits or mates into corresponding cavities formed on the perfusion catheter (12) such that the dilatator (13) can be detachably fixed in a plurality of parking positions in the longitudinal direction of the perfusion catheter (12). Likewise, the protrusions may be formed on the perfusion catheter (12) rather than the dilatator (13), in which case the cavities may be formed on the dilatator (13). The protrusions may be in the form of a flexible teeth or lug (not shown) which would release a snap fit only after exertion of a certain amount of push or pull force applied by the operator in the longitudinal direction.

The catheter (10) may comprise a further channel (not shown) for mixing therapeutic agents in the blood flow through the distal end (25) of the dilatator (13). The therapeutic agents to be added in the blood flow downstream the stenosis may contain fibrinolytic infusion material aiming to dissolve in-situ microvascular thrombus or another desired agent. The further channel for conveying therapeutic agents to the distal end (25) may be located within the shell of the dilatator (13). Preferably, the cross sectional area of lumen (28) is at least 25% more than cross sectional area of the guide wire (15) so that the desired agents may be conveyed around the guide wire (15) the from the proximal end of the dilatator (13) to its distal end. Preferably, the proximal end of the dilatator is suitable for connection to a conventional syringe for injection of therapeutic agents.

As shown in Fig. 7, the catheter (10) may comprise a pressure sensor (20) located at the distal end of the perfusion catheter (12) such that pressure of the blood flow thought the distal end (25) of the tubular perfusion catheter (12) can be monitored in real time. Alternatively or in combination, the catheter (10) may comprise a Doppler sensor (21) located at the distal end of the perfusion catheter (12) for monitoring the velocity of the blood flow through the distal end (25) of the tubular perfusion catheter (12). The pressure data received from the pressure sensor (20) or the Doppler sensor (21) may be transmitted to the proximal end (24) of the catheter (10) via the pressure sensor line (19) and via the Doppler sensor line (18) located, for instance, in the shell of the perfusion catheter (12).

The present invention also discloses a system comprising a catheter (10), the features of which is disclosed in appended Claim 1, the system further comprising a pressure sensor (20) and/or a Doppler sensor (21), a microcontroller (not shown) for receiving and processing data received from one or more sensors and a screen for displaying the received data. In this way, the operator would, easily and in real-time, control the pressure and velocity of the blood flow downstream the occlusion/stenosis, estimate microvascular resitance and adapt the flow as required.

The present invention also discloses a method for treating an occluded blood vessel, said method comprising the steps of;
- penetrating a guiding wire through the occlusion
- advancing/penetrating a dilatator over the guide wire through the occlusion
- advancing a tubular perfusion catheter through the occlusion such that the dilatator is accommodated in the lumen of the perfusion catheter
- optionally inflating a balloon within the stenosis of the occluded blood vessel,
- moving dilatator longitudinally within the lumen of the tubular perfusion catheter such that a controlled reperfusion is established through perfusion holes which are spaced apart from each other in the longitudinal direction of the tubular perfusion catheter.

The method is particularly useful in limiting microvascular damage which occurs during reperfusion of an occluded artery. Using the inventive catheter, system and method of the present invention, interventions which modulate and control initial reperfusion will be particularly appealing to limit occurrence and magnitude of the edema and IMH. Advantageously, controlled pressure or gradual reperfusion of the e.g. epicardial occlusion, with the aim of preventing an abrupt increase in distal intracoronary and consequently capillary hydrostatic pressures during the early reperfusion phase by giving time to arteriolar sphincters until their autoregulatory vasoconstrictor response gets recovered may help to limit leakage from the capillaries and hence may substantially reduce edema and IMH formation.

## Claims

1. A reperfusion catheter (10) for controlled reperfusion in an occluded artery, said catheter (10) comprising;
• a tubular perfusion catheter (12) having a proximal end (24) and a distal end (25) and a lumen (26) defined in between said proximal and distal ends (24,25),
• a dilatator (13) which is sized and shaped to move telescopically in the lumen (26) of said perfusion catheter (12),
**characterized in that**
the tubular perfusion catheter (12) has at least one perfusion port (16) which is formed in the shell of the tubular perfusion catheter (12) and which establishes fluid communication in between the proximal end (24) and the lumen (26) of the perfusion catheter (12),
wherein said at least one perfusion port (16) is fully blocked by the dilatator (13) when said dilatator is fully inserted in the lumen (26) of the perfusion catheter (12),
wherein said at least one perfusion port (16) extends in the longitudinal direction of the perfusion catheter (12) such that, in use, full or partial withdrawal of the dilatator (13) out of the lumen (26) of the perfusion catheter (12) permits controlled flow of blood through said perfusion port (16) and out of the distal end (25) of the tubular perfusion catheter (12).

2. A reperfusion catheter as set forth in Claim 1 wherein said perfusion port (16) is formed by a plurality of perfusion holes (16a) which are spaced apart from each other in the longitudinal direction of the tubular perfusion catheter (12).

3. A reperfusion catheter as set forth in Claim 1 wherein said perfusion port (16) is formed by one or more slits (16b) which extend parallel to the longitudinal direction of the tubular perfusion catheter (12).

4. A reperfusion catheter as set forth in Claim 1 wherein the reperfusion catheter (10) comprises an inflatable balloon (14) wrapped around the outer periphery of the perfusion catheter (12) near its distal end.

5. A reperfusion catheter as set forth in Claim 4 wherein the reperfusion catheter (10) comprises a balloon channel (17) for inflating or deflating the balloon (14).

6. A reperfusion catheter as set forth in Claim 1 wherein which the dilatator (13) has a lumen (28) which connects its proximal and distal ends and which accommodates a guide wire (15).

7. A reperfusion catheter as set forth in Claim 6 wherein the cross sectional area of lumen (28) is at least 25% more than cross sectional area of the guide wire (15) so that an injectate may be conveyed around the guide wire (15) from the proximal end of the dilatator (13) to its distal end.

8. A reperfusion catheter as set forth in Claim 1 wherein the reperfusion catheter (10) comprises a pressure sensor (20) located at the distal end of the perfusion catheter (12) such that pressure of the blood flow thought the distal end (25) of the tubular perfusion catheter (12) can be monitored in real time.

9. A reperfusion catheter as set forth in Claim 1 wherein the reperfusion catheter (10) comprises a Doppler sensor (21) located at the distal end of the perfusion catheter (12) such that velocity of the blood flow thought the distal end (25) of the tubular perfusion catheter (12) can be monitored.

10. A reperfusion catheter as set forth in Claim 1 wherein said dilatator (13) and/or said reperfusion catheter (12), have parking markers (22) for indicating the longitudinal position of the dilatator (13) within the lumen (26) of the perfusion catheter (12).

11. A reperfusion catheter as set forth in Claim 1 wherein the reperfusion catheter (10) comprises a pressure sensor line (19) for conveying data obtained from the pressure sensor (20).

12. A reperfusion catheter as set forth in Claim 1 wherein the reperfusion catheter (10) comprises a Doppler sensor line (19) for conveying data obtained from the Doppler sensor (20).

13. A reperfusion catheter as set forth in Claim 1 wherein the dilatator (13) has one or more flexible protrusions which fits into cavities formed on the perfusion catheter (12) such that the dilatator (13) can be detachably fixed in a plurality of parking positions in the longitudinal direction of the perfusion catheter (12).

14. A system comprising the reperfusion catheter (10) of Claim 1, a pressure sensor (20) and/or a Doppler sensor (21), a microcontroller for receiving and processing data received from one or more sensors and a screen for displaying received data.

15. A method for treating an occluded blood vessel, said method comprising the steps of;
• penetrating a guiding wire (15) through the occlusion
• advancing a dilatator (13) over the guide wire (15) through the occlusion
• advancing a tubular perfusion catheter (12) through the occlusion such that the dilatator (13) is accommodated in the lumen (26) of the perfusion catheter (12)
• optionally inflating a balloon (14) within the stenosis of the occluded blood vessel,
• moving dilatator (13) longitudinally within the lumen (26) of the tubular perfusion catheter (12) such that a controlled reperfusion is established through perfusion holes (16a) which are spaced apart from each other in the longitudinal direction of the tubular perfusion catheter (12).
